(19) Europäisches Patentamt
European Patent Office
Office européen des brevets

(11) **EP 2 538 360 A1**

(12) **EUROPEAN PATENT APPLICATION**

(43) Date of publication:
**26.12.2012 Bulletin 2012/52**

(51) Int Cl.:
***G06F 19/00*** *(2011.01)*

(21) Application number: **11188547.1**

(22) Date of filing: **10.11.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.10.2011 US 552119 P**
**16.06.2011 EP 11170089**

(71) Applicant: **Koninklijke Philips Electronics N.V.**
**5621 BA Eindhoven (NL)**

(72) Inventor: **The designation of the inventor has not yet been filed**

(74) Representative: **Jungen, Carmen**
**Philips Intellectual Property &**
**Standards GmbH**
**Weißhausstraße 2**
**52066 Aachen (DE)**

(54) **Method of predicting a blood dilution risk value**

(57) The present invention provides for a clinical decision support system which makes use of a numerical model which dynamically describes a blood dilution of a blood circulation. Based on measured coagulation data and maybe other patient information, loss of hemostatic balance is predicted based on calculations of certain protein concentrations. Additionally, a calculation arrangement translates at least some of the calculated values of concentrations of human blood proteins into a risk value which risk value describes a risk of clotting and/or embolism and/or bleeding. A time development of that risk value is displayed to the user.

Fig. 1

EP 2 538 360 A1

**Description**

FIELD OF THE INVENTION

The present invention relates to clinical decision support systems. In detail, the present invention relates to a method of predicting a blood dilution risk value of a first blood circulation, to a clinical decision support system for predicting and displaying a blood dilution risk value, a program element for predicting and displaying a blood dilution risk value and a computer readable medium.

BACKGROUND OF THE INVENTION

Patients who undergo surgery experience blood loss and lowered concentrations of blood clotting proteins due to reactions of the hemostatic system to the surgical cuts. Apart from this, the patient's clotting system may be inhibited by Heparin-like compounds to prevent embolism. The blood loss and lowering of coagulation protein concentrations is countered by transfusions of blood plasma, IV fluids, protein substitution solutions etc. The challenge of maintaining a safe hemostatic balance in a patient undergoing surgery is a very difficult one, and multiple strategies exist to meet this challenge.

The patient's coagulation state is monitored during surgery through tests like hematocrit measurements, blood pressure measurements and multiple coagulation tests (e.g. thrombo-elastometry, INR, aPTT). The amounts of (blood) products administered to the patient are of course monitored as well. In current practice the monitored values indicate when the patient is out of hemostatic balance (e.g. his clotting potential has become dangerously low), upon which a countermeasure (e.g. administration of protein substitutes) is put into effect.

A downside to the way of working as described above is that countermeasures are only taken when the patient is already out of balance, and stopped only when the hemostatic balance starts to tip in the other direction.

SUMMARY OF THE INVENTION

It may be seen as an object of the present invention to provide for an improved blood dilution analysis.

There may be a need to analyze blood loss and lowered concentrations of blood clotting proteins and provide accurate countermeasures before the analyzed blood circulation is out of balance. Furthermore, there may be a need to provide a user with the information when the countermeasures have to be stopped and to provide the user with said information before the hemostatic balance of the analyzed blood circulation starts to tip in the other direction.

The present invention matches these needs.

The object of the present invention is solved by the subject-matter of the independent claims. Further embodiments and further advantages are incorporated in the dependent claims.

It should be noted that the embodiments of the invention described in the following similarly pertain to the method, the system, to the program element, as well as to the computer readable medium. In other words, features that will be described with regard to the embodiments relating to a method of predicting blood dilution risk value of a first blood circulation shall be understood to be comprised or implemented by the corresponding system, the program element, and the computer readable medium of the present invention, and vice versa. Especially, the clinical decision support system according to the present invention can be configured in such a way that all the below described method embodiments of the present invention can be carried out by said clinical decision support system.

According to an exemplary embodiment of the invention, a method of predicting blood dilution risk value of a first blood circulation is presented. The method comprises the step of providing measured coagulation data describing a hemostatic situation of the first blood circulation at a first point in time and applying the measured coagulation data as an input for a numerical model. The numerical model is defined to be a mathematical and dynamical representation of a blood dilution of the first blood circulation. In other words, the numerical model describes a blood dilution situation or a blood dilution development of the surveyed or analyzed blood circulation. The method further comprises the step of performing a simulation of a time development of the hemostatic situation by means of the numerical model and based on the measured coagulation data used as an input for the numerical model. Further, calculating values of concentrations of human blood proteins as an output of the simulation is performed. Furthermore, translating at least some of the calculated values of concentrations of human blood proteins into a risk value, which risk value describes a risk of clotting and/or embolism and/or bleeding of the first blood circulation is performed by the presented method.

In other words, the method is configured to provide for a calculated risk value based on measured coagulation data, and moreover the method is additionally configured to provide for a predicted risk value based on a simulation by the numerical model.

In other words, the numerical model used herein describes a situation in which a blood circulation undergoes blood dilution due to for example blood loss or lowered concentrations of blood clotting proteins because of reactions of the hemostatic system due to e.g. a cut.

Furthermore, the term "calculating protein concentrations" may also be understood in the context of the present invention as calculating a concentration of a complex which is formed by at least two different proteins or as calculating a mass-length ratio of a protein, like for example a fibrin fiber.

The step of providing measured coagulation data may for example be embodied by entering a number of transfusion units, that have been supplied to a blood circulation to a graphical user interface, or may be embodied as providing a measured test value of a test which was applied to the blood circulation like e.g. an INR, aPTT, thrombo-elastometry (ampl) or thrombo-elastometry (lag time) test value to a graphical user interface. Such a graphical user interface may be connected with or comprised of a clinical decision support system which performs the presented method. The user may submit the previously and exemplarily mentioned data to a calculation arrangement to allow to perform a corresponding simulation as described above and in the following.

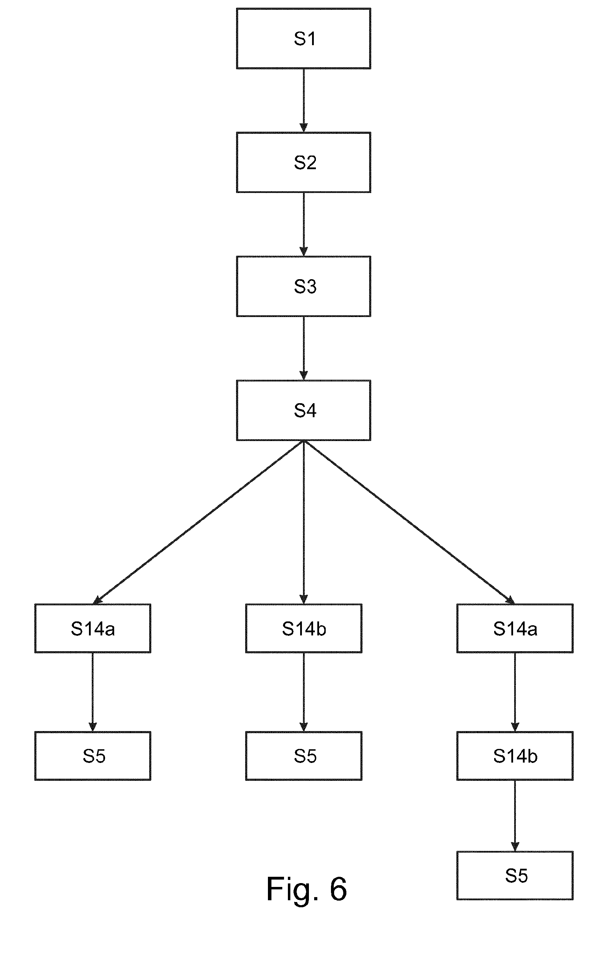

Furthermore the step of "translating" may be performed based on given translation rules and may be performed with regard to a desired specific embodiment of a risk value, like for example a risk value which indicates the speed of sealing of a hypothetical wound. This will be explained in more detail in the following, e.g. with regard to Fig.6.

Furthermore, the term "hemostatic situation" may be understood to be used synonymously to a blood dilution state of the blood circulation. In other words the herein presented numerical model enables a user to calculate and predict the present and future blood dilution states of the analyzed blood circulation and translates the latter into a risk value, i.e. a blood dilution risk value.

Furthermore the term "at least some of the calculated values" shall be understood in the context of the present invention that also only one value of concentration of a human blood protein can be translated if desired. However, a plurality of concentrations of human blood proteins is comprised by said term.

Specifically the term "human blood protein" may be understood in the present invention to also comprise coagulation proteins.

In general, the risk value may be seen as a blood dilution risk value. The risk value may be embodied as a value ranging between 0 and 1 or may be embodied as a displayed color that may have different nuances with respect to the present underlying predicted risk. However, other different representations of the risk value shall be comprised in the scope of the present invention.

In this and every other exemplary embodiment the term "calculating" may be understood as performing a simulation with a model e.g. with the mathematical model described herein.

It may be of importance that the presented method for performing the prediction does not require any contact with the patient, as it is purely based on a mathematical model.

In other words the presented method makes use of a computer model, based on a biochemical model, which will be explained with certain embodiments hereinafter. The model may use biomedical knowledge and experiments, may use the measured monitored test values, i.e. the provided coagulation data like e.g. the above presented number of transfusion units, and other patient information to predict a state of blood dilution that involves a risk of clotting and/or embolism and/or bleeding before it occurs. In other words, when transfusion is given, blood will be diluted. What is predicted by the present invention is whether a patient will become at risk as a result of the dilution.

Such a predicted dangerous state of dilution may be displayed to the user by the calculated and predicted blood dilution risk value, which was generated by the model based on the calculated and predicted blood protein concentrations. Furthermore, the method may comprise to calculate the correspondingly expected effect of each available countermeasure. The solution may be offered to a user by a graphical user interface which interface may be linked to a hospital information system. Therefore, the presented method provides for a continuous estimation of the patients stability, and if desired, an alarm when the patient threatens to become unstable and may suggest for the optimal countermeasure.

According to another exemplary embodiment of the present invention the method comprises the steps of calculating a first predicted risk value based on an assumed first countermeasure, calculating a second predicted risk value based on an assumed second countermeasure, comparing the first and second predicted risk value, and recommending the countermeasure from the first and the second countermeasure which provides for the lower risk value.

According to another exemplary embodiment of the present invention, the calculation of the values of the concentrations of the human blood proteins is generating a predicted time development of said concentrations of human blood proteins as the output of the simulation.

In a further step a generated time development of said concentrations may be translated into a time development of the risk value, which may additionally be graphically displayed to a user. In other words, the presented embodiment may use predicted time series of coagulation proteins for the translation into a risk value.

According to another exemplary embodiment of the invention, the calculated values of the concentrations of human blood proteins are m values of k different proteins, and the method further comprises the step of choosing n values out of the m values. Therein k, m and n are integers and n and m relate to each other as follows: n < m. Furthermore only the n values are taken into account for the translation into the risk value.

In other words the exemplary embodiment presented above may automatically choose only a subset of the calculated and predicted concentrations of the human blood proteins for the further processing into the risk value. Thus, a fast and accurate way of calculating a risk value is presented.

Certain criteria may be given in order to define thresholds, defining whether a protein value is chosen or not. Such thresholds may be stored in e.g. a database and the presented method may compare the calculated concentrations of the human blood proteins with the values retrieved from that database.

Furthermore, more than one concentration value of each different protein may be calculated. In this case the following would be true: m > k.

Additionally, a time development of each concentration of each protein may be calculated and predicted. Depending on said development n values out of the m values may then be chosen.

However, in each of the above identified cases for m, n and k of the present exemplary embodiment, only the n values are taken into a count for predicting and calculating the risk value.

According to another exemplary embodiment of the invention, the method comprises the step graphically displaying a time development of the risk value on a graphical user interface.

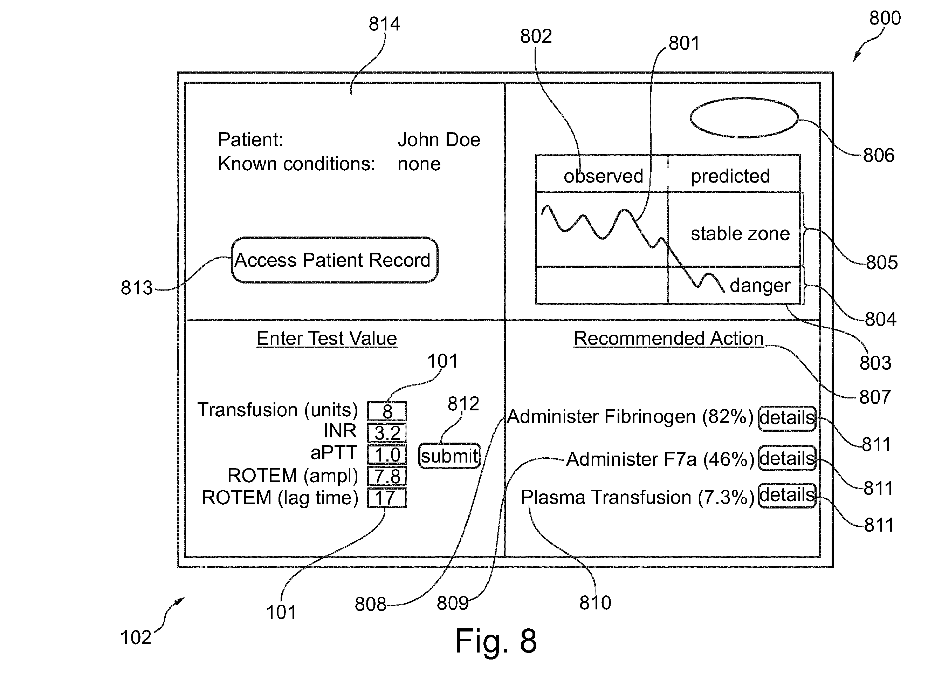

As can be gathered for example from Fig. 8, this embodiment provides for the advantage for a user to be able to gather information in an illustrative way about the future development of the risk value. Hence, a fast and reliable decision can be made by the user observing the graphical representation of the time development of the risk value. Thus, according to another exemplary embodiment of the invention, the method may comprise the step of calculating a time development of the blood dilution a risk value during or besides the translation. If desired, such a time development of a risk value may be displayed in a x- and y-diagram, wherein the x-axes depicts the time and the y-axes depicts the blood dilution risk value of clotting and/or embolism and/or bleeding of the blood circulation. In other words the risk value is shown as a function of time.

According to another exemplary embodiment, the at least some of the values of the concentrations of the human blood proteins are translated into a risk value by means of a numerical function of state variables of the numerical model.

Furthermore, said state variables may be used to describe or define a danger zone or a danger level regarding the blood dilution. Such state variables may be embodied for example as a concentration of proteins that play a role in the coagulation process. Such a danger level or patient stability score may be implemented as an aggregate variable which is the numerical function. The numerical model may be used to identify the set of most sensitive state variables in the situation of blood dilution, i. e. those protein concentrations that at a small increase or decrease of their value, determine whether a coagulation response is too strong, i. e. a risk of embolism, or too weak, i. e. risk of excessive bleeding. Different state variables may be related to the two different risks, and some strong influences on risk may be due to a combination of multiple parameters. Furthermore, model analysis methods can be used for such a sensitivity analysis and can be used to identify a panel of state variables. Furthermore, a more exact definition the numerical function can be estimated through a clinical study where the levels of the related protein concentrations are measured in case of bleeding and/or embolism. In other words the use of the numerical model in the presented embodiment is two-fold. Firstly for the identification of the panel of state variables, and secondly to predict the expected development of the dynamic values of these state variables and thus the risk value during a situation of blood dilution.

According to another exemplary embodiment, the state variables of the numerical model are chosen from the group comprising concentrations of the following proteins: Alpha-2-Macroglobulin (A2M), C4BP, coagulation factor 10 (F 10), F11, F13, prothrombin (F2), tissue factor, F5, F7, F8, F9, fibrinogen, fibrin, protein C, protein S, protein Z, protein Z related protein inhibitor (ZPI), alpha-1-anti-trypsin (AAT), protein C inhibitor (PCI), anti-thrombin (ATIII), PAI1, C1 inhibitor (C1inh), TAFI, TFPI, Vitronectin, plasmin, plasminogen, A2AP, thrombomodulin, uPA, tPA, the proteins' activated forms F10a, F11a, F13a, thrombin (F2a), F5a, F7a, F8a, F9a, activated protein C, and TAFIa, or wherein the state variables of the numerical model is a concentration of complexes formed by at least two of the previously cited proteins (e.g. FVa-FXa), or wherein the state variables of the numerical model is a mass-length ratio of fibrin fibers formed in coagulation. Furthermore each protein comprises by one of the following Tables 1 to 3 may be state variable according to this embodiment.

According to another exemplary embodiment the method further comprises the step of using the numerical model to identify a set of most sensitive state variables in a situation of blood dilution based on at least one given sensitivity threshold.

The presented embodiment may make use of a comparison between calculated values of the state variables with the sensitivity threshold.

Sensitive state variables can be identified through a sensitivity analysis method, which involves the variation of one or a set of model parameters or model inputs and the analysis of the change in one or more model outputs as a result of the change in the model parameters or model input. The case of sensitive state variable selection in a hemostatic model involves the simulation of a clotting response to a certain trigger, e.g. exposure of proteins residing in blood to a wound in the blood vessel wall, and more specifically to the tissue factor protein at the wound surface. The varied model inputs are the initial values for the protein concentrations state variable used in the model, whereas the observed output can be any model feature that links to the strength of the clotting response, e.g. the time between first exposure of the blood to tissue factor and the moment that thrombin a key protein that is produced in the clotting process exceeds a threshold value of e.g. 10 nM.

In the simplest form of sensitivity analysis one model input is varied within its theoretical limits while the other model inputs are kept constant, i.e. local sensitivity analysis. The resulting change in model output may be translated to a sensitivity score, i.e. single value, for the one varied model input, where this score will be low if the model output changes little with the varying model input and high when the output changes strongly as a result of the varying model input. Such a score may also depend on the correlation between the change in the model input and the change in the model output, i.e. a model output that rises consistently with a rising model input may lead to a higher sensitivity score than a model output that changes strongly, but erratically with a rising model input. Local sensitivity analysis calculates one sensitivity score for each model input relating to a state variable; the highest sensitivity scores identify the most sensitive state variables.

In the more complicated global sensitivity analysis all model inputs e.g. initial values for the state variables are changed simultaneously. This method is less sensitive to the choice of fixed model inputs (in the local sensitivity analysis all model inputs but one are fixed to a chosen value), but has the downside that the response of the chosen model output to the change in a chosen model input is influenced by the variation of all the other model inputs. This makes the variation of the model output as a function of the variation of one model input more chaotic by definition. Sensitivity scores can still be calculated, e.g. as the correlation coefficient between a model input and the selected model output (see also Frey, H.C., Patil, S. R., Identification and Review of Sensitivity Analysis Methods. Risk Anal., 2002. 22(3): p. 553-578.)

According to another exemplary embodiment of the invention, the method further comprises the step of providing data about human blood protein levels of a second blood circulation at a second point in time as reference protein levels. Therein, at the second point in time the second blood circulation undergoes bleeding and/or clotting and/or embolism, wherein the second point in time is before the first point in time. Furthermore, the embodiment comprises using said provided reference protein levels for the translation into the risk value for the first blood circulation.

Thus, the first blood circulation may be different from the second blood circulation, as the reference protein levels may usually be used from different patients. In other words, this embodiment describes the situation of the usage of previously gathered information, how blood circulations and coagulations systems may react in average during blood dilution. Said provided data may be retrieved by a clinical decision support system from internal or external data storage, where corresponding average protein levels during blood dilution are stored. By means of comparing values of the same protein, an estimation of the numerical function is provided.

This embodiment may also be used to provide for a patient-specific observation during surgery. In this case the first blood circulation and the second blood circulation are the same. Thus, if desired, as reference protein levels data may be used of the first blood circulation, i. e. data from the same patient, from a previous point in time, at which the patient was undergoing bleeding and/or clotting and/or embolism or which may be related to a bleeding and/or clotting and/or embolism event at a later point in time. Consequently, the presented method makes use of knowledge, how the protein level of the specific observed patient is developing under said situations.

According to another exemplary embodiment of the invention, the method further comprises the step of comparing said provided reference protein levels with the calculated values of concentration of human blood proteins, generating a comparison value and using the comparison value for the translation into the risk value for the first blood circulation.

According to another exemplary embodiment of the invention, the at least some of the values of the concentration of the human blood proteins are translated into the risk value by calculating a speed of sealing of a hypothetical wound or by calculating a speed of growth of a hypothetical thrombus as an output.

In other words, the presented embodiment involves a prediction of certain clot elements, like for example fibrin or fibrinogen concentration. Furthermore, the increase of fiber which comprises or consists of fibrin or fibrinogen may be calculated. Based on the previously cited steps, the presented method may translate this into a wound closing time that is necessary to close the hypothetical wound. The presented method may further translate the wound closing time into a corresponding bleeding risk value.

In a similar way based on the calculated concentration of proteins, a size of a thrombus may be calculated. This may further be related to an estimated wound closing. In other words, the numerical model may describe how fast a thrombus may grow and thus how fast the sealing of a wound takes place, all based on the provided coagulation data. Both aspects, which are predicted and calculated by the numerical model, i. e. the wound closing time and the growth of a thrombus, are subsequently translated into a corresponding risk value. Thus, the risk value may firstly be embodied as a wound closing time risk value or may secondly be embodied as a thrombus growing risk value. If desired, the calculated and translated risk value may be based on both predicted results, the wound closing time and the growth of a thrombus.

According to another exemplary embodiment, the numerical model is a model of a time development of a hypothetical sealing of a wound of the first blood circulation. Furthermore, this embodiment further comprises the step of performing the simulation of the time development of the hypothetical sealing of the wound of the first blood circulation in terms of at least one element of the group comprising a wound surface area, interaction of tissue factor in a wound surface area with coagulation proteins in the first blood circulation, formation of fibrin fibers, and aggregation of blood platelets and/or fibrin fibers, which cover a wound surface and stop a clotting process. In other words, the variables that are simulated are of the group comprising the before cited variables.

Thus the numerical model takes into account at least one of the above cited elements in order to calculate as an output a wound closing time. This wound closing time may then be translated, as described above, into a risk value, which may then be graphically displayed to a user.

According to another exemplary embodiment, the method further comprises the step of evaluating the simulated time development of the hypothetical sealing of the wound by evaluating a time that passes between an initialization of the clotting process, i. e. a formation of the wound, and a cessation of the clotting process, i. e. a sealing of the wound. Then a risk value may be calculated, namely a bleeding risk value.

According to another exemplary embodiment, the method further comprises the step of evaluating a risk value based on size and constitution of the thrombus. This may be risk value regarding clotting or embolism.

According to another exemplary embodiment of the invention, a clinical decision support system for predicting and displaying a blood dilution risk value of a first blood circulation is presented wherein the system comprises a first arrangement configured to receive measured coagulation data describing a hemostatic situation of the first blood circulation at a first point in time. The system further comprises a storing arrangement on which a numerical model is stored, wherein the numerical model is a mathematical and dynamical representation of a blood dilution of the first blood circulation. The system further comprises a calculation arrangement configured to perform a simulation of a time development of the hemostatic situation by means of the numerical model and based on the measured coagulation data used as an input for the numerical model. Therein, the calculation arrangement is configured to calculate values of concentration of human blood proteins as an output of the simulation. Furthermore, the calculation arrangement is configured to translate at least some of the calculated values of concentrations of the human blood proteins into a risk value, which risk value describes of risk of clotting and/or embolism and/or bleeding of the first blood circulation. Furthermore, the system further comprises a display arrangement configured to display the risk value.

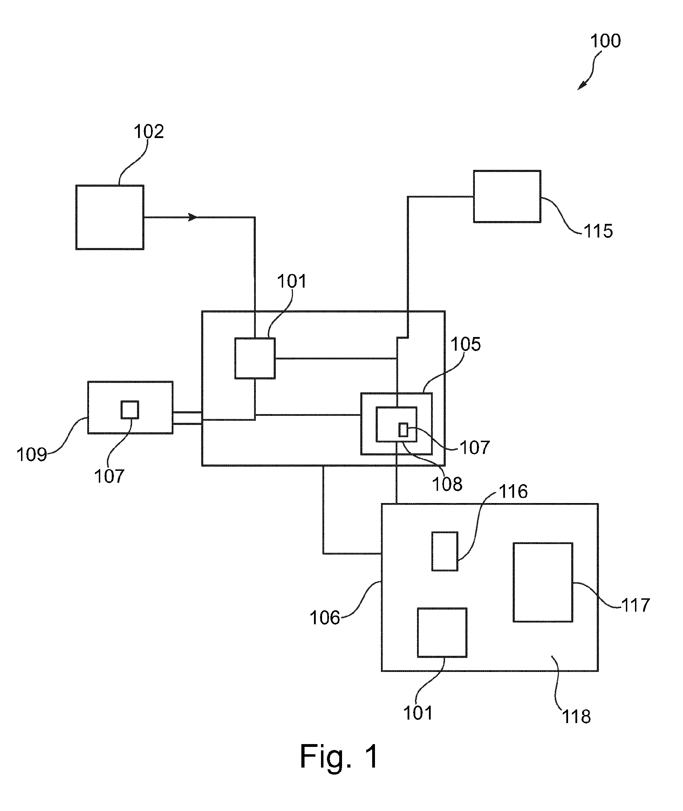

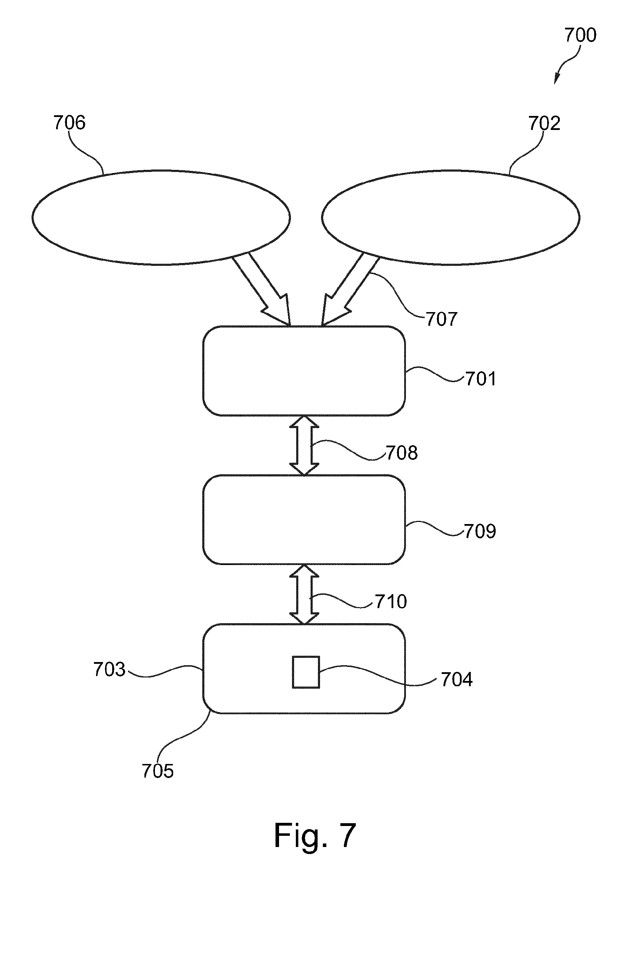

In addition to the previously described configurations of that clinical decision support system, this clinical decision support system may also be configured to perform the described methods as presented above and in the following. Such a clinical decision support system is depicted in the following Figs. 1 and 7.

The presented clinical decision support system makes use of a numerical model which may be based on biochemical knowledge and/or experiments, which uses the provided calculation data. If desired other patient information may also be used to predict a blood dilution risk value in future. Based on calculated future protein concentrations, a risk value is calculated by the system and graphically displayed to the user in order to provide him with the necessary information before loss of hemostatic balance occurs. Furthermore, the system may be configured to calculate an effect of different countermeasures that may be performed by the user. The clinical decision support system may be linked to a hospital information system and provides for a continuous estimation of the patient's stability. If desired, an alarm when the patient threatens to become unstable may be provided to the user. Additionally one or more suggestions of optimal countermeasures may be applied to the user by the system. The suggestions may be ranked based on the predicted chances of success.

According to another exemplary embodiment of the invention, a program element for predicting and displaying a blood dilution risk value of a first blood circulation, which when being executed by a processor is adapted to carry out steps of receiving measured coagulation data describing the hemostatic situation of the first blood circulation at a first point in time, applying the measured coagulation data as an input for a numerical model, the numerical model being a mathematical and dynamical representation of a blood dilution of the first blood circulation, and performing a simulation of a time development of the hemostatic situation by means of the numerical model, and based on the measured coagulation data used as an input for the numerical model, and calculating values of concentrations of human blood proteins as an output of the simulation, and translating at least some of the calculated values of concentrations of human blood proteins into a risk value, which risk value describes a risk of clotting and/or embolism and/or bleeding for the first blood circulation.

The computer program element may be part of a computer program, but it can also be an entire program by itself. For example, the computer program element may be used to update an already existing computer program to get to the present invention.

According to another exemplary embodiment, a computer readable medium in which a program element for predicting and displaying a blood dilution risk value of a first blood circulation is stored, which, when being executed by a processor, is adapted to carry out the steps of receiving measured coagulation data describing the hemostatic situation of the first blood circulation at a first point in time, and applying the measured coagulation data as an input for a numerical model, the numerical model being a mathematical and dynamical representation of a blood dilution of the first blood circulation, and performing a simulation of a time development of the hemostatic situation by means of the numerical model and based on the measured coagulation data used as an input for the numerical model, and calculating values of concentrations of human blood proteins as an output of the simulation, and translating at least some of the calculated values of concentrations of human blood proteins into a risk value, which risk value describes a risk of clotting and/or embolism and/or bleeding for the first blood circulation.

The computer readable medium, as for example shown in Fig. 1, may be seen as a storage medium, such as for example, a USB stick, a CD, a DVD, a data storage device, a hard disk, or any other medium on which a program element as described above can be stored.

It may be seen as a gist of the invention to use a numerical model, which is a mathematical and dynamical representation of a situation of blood dilution of the blood circulation, to predict future protein concentrations of said blood circulation based on the received coagulation data. Time developments of said protein concentrations may thus be calculated and predicted by the model. Based on said predicted protein concentrations an assessment of the participating proteins is performed by means of which the most relevant proteins are chosen. The group of chosen proteins is used to calculate and predict a blood dilution risk value, and to show said value or a time development of said value to a user. Corresponding recommendations for countermeasures may be predicted and displayed to the user, somehow rated with regard to the respective chances of success. In other words, the invention is able to provide for a present risk value based on measured coagulation data, and the invention is additionally able to provide for a predicted risk value based on simulation.

Furthermore, a person skilled in the art will gather from the above and the following description that, unless otherwise notified, in addition to any combination belonging to one type of subject-matter, also any combination between features relating to different subject-matters, in particular between features of the apparatus type claims and features of the method type claims, is considered to be disclosed with this application. Furthermore, all features can be combined providing synergetic effects that are more than the simple summation of the features.

The present invention will become apparent from and be elucidated with reference to the embodiments described hereinafter.

Exemplary embodiments of the invention will be described in the following drawings.

BRIEF DESCRIPTION OF THE DRAWINGS

Fig. 1        schematically shows a clinical decision support system according to an exemplary embodiment of the invention.

Figs. 2 to 6  schematically show flow diagrams of a method according to exemplary embodiments of the invention.

Fig. 7        schematically shows a clinical decision support system according to an exemplary embodiment of the invention.

Fig. 8        schematically shows a graphical user interface to be used in accordance with an exemplary embodiment of the invention.

In principal, identical parts are provided with the same reference symbols in the figures.

DETAILED DESCRIPTION OF EMBODIMENTS

Fig. 1 shows a clinical decision support system 100 for calculating and displaying a prediction of a blood dilution risk value a first blood circulation according to an exemplary embodiment of the invention. The system 100 comprises a first arrangement 101 configured to receive measured coagulation data 102 which data describe a hemostatic situation of the first blood circulation at a first point in time. The system 100 further comprises a storing arrangement 103 on which a numerical model 104 is stored. The numerical model is embodied as a mathematical and dynamical representation of a blood dilution situation of the first blood circulation. The calculation arrangement 105 is configured to perform a simulation of a time development of the hemostatic situation by means of using the numerical model and supplying the measured coagulation data as an input to that model. Furthermore, the calculation arrangement 105 is configured to calculate values of concentrations of human blood proteins as an output of the simulation. Furthermore, the calculation arrangement 105 is configured to translate at least some of the calculated values of concentrations of human blood proteins into a risk value which describes a clotting and/or embolism and/or bleeding of the first blood circulation. Furthermore, a display arrangement 106 is shown in Fig. 1. By means of the display arrangement the calculated risk value can be shown to the user. Additionally, a program element 107 is shown in Fig. 1 which is configured for calculating and displaying a prediction of a hemostatic situation of a first blood circulation, when being executed by the processor 108. As can be gathered from Fig. 1, a computer readable medium 109 is shown on which such a program element 107 is additionally stored. Moreover, a data storage device 115 is shown which is linked to the present clinical decision support system. For example, the data storage device 115 may be embodied as a content information system or a content delivery system (CDS). The display arrangement 106 provides for a graphical user interface 118 which has an area 116 where the predicted risk value, which has been calculated by means of the previously described translation, is displayed. If desired, a time development of that risk value is displayed there to the user. As can be seen in Fig. 1, the graphical user interface additionally provides for a first arrangement 101 which is configured to receive measured coagulation data. Fig. 8 depicts an embodiment of a graphical user interface that could be used as user interface 118 of Fig. 1. In combination with Fig. 8, where also a first arrangement 101 configured to receive measured coagulation data is shown, it becomes clear that information about e. g. already supplied transfusion units or INR or aPTT test values may be entered and submitted to the clinical decision support system by the user. Additionally, the graphical user interface 118 comprises an area 117 at which recommendations for countermeasures can be displayed to the user, which countermeasures were calculated previously by the clinical decision support system. These recommended countermeasures are based on the model predictions as described above and in the following.

In other words, said exemplary embodiment may be seen as a clinical decision support system performing a computer-supported decision method. By means of the calculated predictions a user may decide which administration of drugs or other clinical action may be useful. The result of the prediction may be accompanied by a calculated suggestion of a change of administration of anti- or pro-coagulation drugs. Said calculation of the prediction may be performed e.g. on a computer or a processor of a computer.

A gist of this mathematical model can be seen in the combination of a biochemical model calculating coagulation cascade and fibrin polymerization. Thus, "enzymatic conversion" in combination with "complex assembly" can be taken into account during the prediction.

The mathematical model used in Fig. 1 will be explained in more detail hereinafter. This model may be implemented in every herein described embodiment of the invention. It is of utmost importance that the below presented model is just one exemplary embodiment of the mathematical model according to the present invention.

It should be noted that the mathematical model can, if desired, comprise partially or completely the reaction mechanisms that are disclosed in Tables 1 to 3. Also the ordinary differential equations disclosed in Tables 4 and 5 may be integrated into the mathematical model according to the user's desire. In other words, also a combination between different reaction mechanisms out of Tables 1 to 3 with ordinary differential equations out of Tables 4 and 5 are possible. In other words the person skilled in the art may take from the presented tables 1 to 5 the features he is interested in regarding his special medical case. Thus, it is made clear that the model presented herein is just a version of the model, and that this model can be adapted, extended, reduced or even completely replaced by another mathematical model which takes into account biochemical and pharmacodynamical aspects.

**Mathematical Definition of the Model**

The mathematical model can be considered to consist of three separate modules: coagulation cascade, fibrin polymerization and pharmacokinetics and pharmacodynamics (PK/PD) of anticoagulant drugs. If desired only the first two modules may be used. Whereas the first two modules are based on the underlying (protein) interactions of the coagulation response and may be used to simulate in vitro tests like the thrombin generation assay, prothrombin time (PT) and activated partial thromboplastin time (aPTT), the latter is based on compartment modeling which is used to simulate the (long-term) kinetics and effect of the anticoagulants such as unfractionated heparin (UFH) and low-molecular weight heparin (LMWH) in the human body.

**Biochemical Model**

The physiological system of biochemical reactions may be represented as a closed volume element, representing a certain volume of blood plasma in in vitro tests. Hence, there is no transport in or out of this volume and clearance of proteins is assumed to be not significant on this time-scale (minutes). This means that there is conservation of mass in the volume element. Besides that it is assumed that diffusion in the mixture does not significantly influence the reaction velocities.

The mathematical model of the coagulation cascade and fibrin polymerization consists of 216 state variables (concentrations of proteins and protein complexes) and 100+ reaction rate constants that are used to parameterize 91 reactions. An overview of the reactions is given in Table 1, Table 2 and Table 3. All states, initial concentrations and kinetic parameters were defined as non-negative real numbers, IR+0. The initial concentrations of the proteins are inferred from values reported in literature or set to the actual measured concentrations. The model's kinetic parameters were estimated from in-house generated experimental data by means of solving the inverse problem. Nevertheless, the kinetic parameters (but also the initial concentrations) are subjected to a continuous update process to improve the accuracy of the found values by means of additional experiments and analyses.

The functional description of the state equations can be represented as follows (in state space formulation).

$$\frac{dx}{dt} = f(x(t), u(t), \theta), \text{ with } x_0 = x(t_0) \tag{1}$$

Where x is the state vector, u the input vector of the test conditions (e.g. certain tissue factor concentration to simulate the PT), x0 is the vector of initial concentrations and f is a vector field with non-linear functions parameterized with $\theta$. The output, y, of the state model can be characterized by:

$$y(t, \theta) = \underline{C} \, x(t, \theta) \tag{2}$$

Where matrix C selects a number of 'interesting' states of the model output.

The 91 reaction mechanisms derived from literature were classified as either one of two types of elementary reaction mechanisms. These reaction mechanisms were complex assembly and enzymatic conversion.

Complex assembly is the process where substrate A and B react to form complex A-B. It features in the formation of coagulation complexes (e.g. FXa-FVa, FIXa-FVIIIa) and inhibition of activated proteins by stochiometric inhibitors (e.g. FIIa-AT-III, TF-FVIIa-FXa-TFPI). The related reaction equation reads:

$$A + B \underset{k_{-1}}{\overset{k_1}{\rightleftarrows}} A - B \tag{3}$$

The association rate constant of complex formation, k1, is a second order rate constant and the dissociation rate constant of A and B from A-B, k-1, is a first-order rate constant. In some cases the association reaction is irreversible, which means the complex is stable and will not dissociate, e.g. inhibition of FIIa by AT-III. Reaction scheme (3) was converted to the following set of ordinary differential equations (ODEs) describing the change in concentration, represented by [...], in time:

$$\frac{\partial[A]}{\partial t} = \frac{\partial[B]}{\partial t} = -\frac{\partial[A-B]}{\partial t} = -k_1[A][B] + k_{-1}[A-B] \tag{4}$$

The enzymatic conversion of proteins by enzymes was the second type of reaction mechanism exploited in the coagulation model. All activation processes in the hemostasis model correspond to this type of reaction. The reaction scheme of enzymatic conversion can be represented schematically as:

$$E + S \underset{k_{-1}}{\overset{k_1}{\rightleftarrows}} E - S \overset{k_2}{\rightarrow} E + P \tag{5}$$

Where E is the enzyme and S the substrate concentration that is converted into product P by E. Enzymatic conversion of proteins was implemented in the mathematical model as follows:

$$\frac{\partial[E]}{\partial t} = -k_1[E][S] + k_{-1}[E-S] + k_2[E-S] \tag{6}$$

$$\frac{\partial[S]}{\partial t} = -k_1[E][S] + k_{-1}[E-S] \tag{7}$$

$$\frac{\partial[E-S]}{\partial t}=k_1[E][S]-k_{-1}[E-S]-k_2[E-S] \qquad (8)$$

$$\frac{\partial[P]}{\partial t}=k_2[E-S] \qquad (9)$$

Most of the proteins or protein-complexes participate in multiple reactions in the biochemical model, hence all reactions that the protein or protein-complex is participating in have to be accounted for in the ODE of that specific protein's or protein-complex' concentration. This results in one ODE per protein or protein-complex, which consists of a summation of ODE contributions from all reactions that the protein is participating in. This is represented mathematically as follows (an alternative representation of equation (1)).

$$\frac{\partial x}{\partial t}=\underline{S}\,\underline{R}(x)=\sum_{i=1}^{m}S^i R_i(x) \qquad (10)$$

Where x is the vector of concentrations of the different substrates, S is the matrix with reaction rate constants and R is the reaction matrix. Each column of the stochiometric matrix Si corresponds to a particular reaction.

## PK/PD Model

The mathematical model that simulates the long-term kinetics and effects of the anticoagulants is based on a combination of compartment models that are generally used in PK/PD modeling. Since the PK/PD equations are not as standardized as the biochemical equations (only complex assembly and enzymatic catalysis), the complete ODEs of each state are shown in Table 4 and Table 5. The ODEs belonging to the pharmacokinetic properties of unfractionated heparin and low-molecular weight heparin are shown in Table 4. As a result of these ODEs the blood kinetics of both types of heparin can be calculated. The effects of both heparins are on the activity of AT-III, and this is represented by equations v78 - v91 in the biochemical model, which uses the blood concentrations of UFH and LMWH at the moment of blood withdrawal as input. The blood concentrations of the coagulation proteins at the moment of blood withdrawal are used as input for the biochemical model.

The Tables 1 to 4 are shown in the following. The mathematical model described herein may thus take into account several or all reaction mechanisms v1 to v91. The person skilled in the art will combine them as needed or desired. Additionally the ordinary differential equations described under PKPD1 to PKPD17 may partially or completely be implemented in the mathematical model.

The used model may also be described as follows: The computer model may be seen as a representation of the coagulation cascade and fibrin polymerization as a set of reaction mechanisms. The time dynamics of each reaction mechanism may be described as an ordinary differential equation or ODE that involves the concentration(s) of the protein (s) and/or chemical molecule(s) that are involved in the reaction and the reaction rate parameter(s). By summation of all reaction mechanisms in which a particular protein or other kind of chemical molecular is involved (a protein or molecule can participate in more than one reaction), the time dynamics of the concentration of that particular protein or other kind of chemical entity may be calculated. Doing this for all proteins or molecules, the whole system can calculate and keep track of the evolution of all proteins and molecules over time, however for this one may require, beside the reaction topology, also the numerical values of the model parameters. These model parameters include the initial conditions of the system, i.e. the concentration of all proteins and molecules at t = 0 (e.g. before onset of bridging therapy), and the reaction rate parameters of the reaction mechanisms. Part of the initial concentrations that are the most important to the outcome of the system are measured from the patient (in the laboratory or clinic), whereas others, less determining proteins, are taken from literature (average patient values, possibly corrected for gender and age, etc.). The reaction rate parameters may be derived via solving an inverse problem, i.e. model fitting to experimental data. The system of coupled ODEs may be solved numerically, using the numerical values of the model parameters, by employing standard ODE integration algorithms.

Table 1 All reaction mechanisms incorporated in the computer model of the coagulation cascade. It should be noted that in this table the official gene symbole are used instead of the popular scientific names

| Reaction | Name | Type | Substrates | Products | Cofactors/ Catalyst | Reaction site |
|---|---|---|---|---|---|---|
| v1 | F3-F7a complex assembly | Complex assembly | F3,F7a | F3-F7a | | Endothelial membrane |
| v2 | F3-F7 complex assembly | Complex assembly | F3, F7 | F3-F7 | | Endothelial membrane |
| v3 | F7 activation (1) | Catalysis | F7 | F7a | F3-F7a | Endothelial membrane |
| v4 | F7 activation (2) | Catalysis | F7 | F7a | F10a | Endothelial membrane |
| v5 | F7 activation (3) | Catalysis | F7 | F7a | F9a | Endothelial membrane |
| v6 | F7 activation (4) | Catalysis | F7 | F7a | F2a | |
| v7 | F9 activation (1) | Catalysis | F9 | F9a | F11a, negative phospholipids | |
| v8 | F9 activation (2) | Catalysis | F9 | F9a | F3-F7a | Endothelial membrane |
| v9 | F9a degradation | Degradation | F9a | | | Blood plasma |
| v10 | F8 activation (1) | Catalysis | F8 | F8a | F2a | Blood plasma?? |
| v11 | F8 degradation | Degradation | F8a | | PROCa-PROS1-F5ac | Platelet membrane |
| v12 | F9a-F8a complex assembly | Complex assembly | F9a, F8a | F9a-F8a | Ca2+, neg phospholipid | Platelet membrane |
| v13 | F2 activation (1) | Catalysis | F2 | F2a | F10a | Blood plasma |
| v14 | F2 activation (2) | Catalysis | F2 | F2a | F10a-F5a | Platelet membrane |
| v15 | F2a degradation | Degradation | F2a | | | Blood plasma |
| v16 | F5 activation | Catalysis | F5 | F5a | F2a | Blood plasma |
| v17 | F5 anticoagulant formation | Catalysis | F5 | F5ac | PROCa | Blood plasma |
| v18 | F5a degradation | Degradation | F5a | | PROCa-PROS1 | Blood plasma/ endothelial membrane |
| v19 | F10 activation (1) | Catalysis | F10 | F10a | F3-F7a | Endothelial membrane |
| v20 | F10 actication (2) | Catalysis | F10 | F10a | F9a-F8a | Platelet membrane |
| v21 | F10 activation (3) | Catalysis | F10 | F10a | F9a | Blood plasma?? |
| v22 | F10a degradation | Degradation | F10a | | | Blood plasma |

(continued)

| Reaction | Name | Type | Substrates | Products | Cofactors/ Catalyst | Reaction site |
|---|---|---|---|---|---|---|
| v23 | F10a-F5a complex assembly | Complex assembly | F10a, F5a | F10a-F5a | Ca2+, neg phospholipid | Platelet membrane |
| v24 | PROC activation (1) | Catalysis | PROC | PROCa | F2a | Blood plasma |
| v25 | PROS1-C4BP complex assembly | Complex assembly | PROS1, C4BP | PROS1-C4BP | | Blood plasma |
| v26 | PROCa-PROS1 complex assembly | Complex assembly | PROCa, PROS1 | PROCa-PROS1 | Ca2+, neg phospholipid | Platelet membrane |
| v27 | PROCa-PROS1-F5ac complex assembly | Complex assembly | PROCa-PROS1, F5ac | PROCa-PROS1-F5ac | Ca2+, neg phospholipid | Platelet membrane |
| v28 | F13 activation | Catalysis | F13 | F13a | F2a,Ca2+(at least 1mM) | Blood plasma |
| v29 | F12 activation (1) | Catalysis | F12 | F12a | F12a, negative phospholipds | Negative surface |
| v30 | F12 activation (2) | Catalysis | F12 | F12a | KLKB1a | Blood plasma |
| v31 | F11 activation (3) | Catalysis | F12 | F12a | KNG1 | Blood plasma |
| v32 | F12a degradation | Degradatiion | F12a | | | Blood plasma?? |
| v33 | KLKB1 activation | Catalysis | KLKB1 | KLKB1a | F12a | Blood plasma |
| v34 | F11 activation (1) | Catalysis | F11 | F11a | F12a | Blood plasma |
| v35 | F11 activation (2) | Catalysis | F11 | F11a | F2a, negative phospholipids | Negative surface |
| v36 | F11 activation (3) | Catalysis | F11 | F11a | F11a, negative phospholipids | Negative surface |
| v37 | F11a degradation | Degradation | F11a | | | Blood plasma |
| v38 | CPB2 activation (1) | Catalysis | CPB2 | CPB2a | F2a | Blood plasma |
| v39 | CPB2a degradation | Degradation | CPB2a | | | Blood plasma |
| v40 | F10a-TFPI complex assembly | Complex assembly | TFPI,F10a | F10a-TFPI | | Blood plasma?? |
| v41 | F10a-F3-F7a-TFPI complex assembly | Complex assembly | F10a-TFPI, F3-F7a | F10a-F3-F7a-TFPI | Ca2+ | Endothelial membrane |
| v42 | F3-F7a-TFPI complex assembly | Complex assembly | F3-F7a, TFPI | F3-F7a-TFPI | | Endothelial membrane |

(continued)

| Reaction | Name | Type | Substrates | Products | Cofactors/ Catalyst | Reaction site |
|---|---|---|---|---|---|---|
| v43 | F11a-SERPINC1 complex assembly | Complex assembly | F11a, SERPINC1 | F11a-SERPINC1 | SERPIND1 | Blood plasma |
| v44 | F12a-SERPINC1 complex assembly | Complex assembly | F12a, SERPINC1 | F12a SERPINC1 | SERPIND1 | Blood plasma |
| v45 | F9a-SERPINC1 complex assembly | Complex assembly | F9a, SERPINC1 | F9a-SERPINC1 | SERPIND1 | Blood plasma |
| v46 | F2a-SERPINC1 complex assembly | Complex assembly | F2a, SERPINC1 | F2a-SERPINC1 | SERPIND1 | Blood plasma |
| v47 | F10a-SERPINC1 complex assembly | Complex assembly | F10a, SERPINC1 | F10a-SERPINC1 | SERPIND1 | Blood plasma |
| v48 | F3-F7a-SERPINC1 complex assembly | Complex assembly | F3-F7a, SERPINC1 | F3-F7a-SERPINC1 | SERPIND1 | Blood plasma |
| v49 | PROCa-SERPINA1 complex assembly | Complex assembly | PROCa, SERPINA1 | PROCa-SERPINA1 | | Blood plasma |
| v50 | PROCa-SERPINA5 complex assembly | Complex assembly | PHOCa, SERPINA5 | PROCa-SERPINA5 | Heparin dependent | Blood plasma |
| v51 | F2a-SERPINA5 complex assembly | Complex assembly | F2a, SERPINA5 | F2a-SERPINA5 | Heparin dependent | Blood plasma |
| v52 | F10a-SERPINA5 complex assembly | Complex assembly | F10a, SERPINA5 | F10a-SERPINA5 | Heparin dependent | Blood plasma |
| v53 | KLK1a-SERPINA5 complex assembly | Complex assembly | KLKB1a, SERPINA5 | KLKB1a-SERPINA5 | | Blood plasma?? |
| v54 | PROZ-SERPINA10 complex assembly | Complex assembly | PHOZ, SERPINA10 | PROZ-SERPINA10 | | Blood plasma |
| v55 | F9a-SERPINA10 complex assembly | Complex assembly | F9a, SERPINA10 | F9a-SERPINA10 | | Blood plasma |

(continued)

| Reaction | Name | Type | Substrates | Products | Cofactors/ Catalyst | Reaction site |
|---|---|---|---|---|---|---|
| v56 | F10a-PROZ-SERPINA10 complex assembly | Complex assembly | PROZ-SERPINA10, F10a | F10a-PROZ-SERPINA10 | Ca2+, Phospholipids | Membrane |
| v57 | F11a-SERPINA10 complex assembly | Complex assembly | F11a, SERPINA10 | F11a-SERPINA10 | | Blood plasma |
| v58 | PROCa-SERPINE1 complex assembly | Complex assembly | PROCa, SERPINE1 | PROCa-SERPINE1 | | Blood plasma?? |
| v59 | F2a-SERPINE1 complex assembly | Complex assembly | F2a, SERPINE1 | F2a-SERPINE1 | | Blood plasma |
| v60 | VTN-SERPINE1 complex assembly | Complex assembly | VTN, SERPINE1 | VTN-SERPINE1 | | Membrane surface |
| v61 | F2a-VTN-SERPINE1 complex assembly | Complex assembly | F2a, VTN-SERPINE1 | F2a-VTN-SERPINE1 | | Membrane surface |
| v62 | CPB2a-SERPINE1 complex assembly | Complex assembly | CPB2a, SERPME1 | CPB2a-SERPINE1 | | Blood plasma |
| v63 | SERPINE1 degradation | Degradation | SERPINE1 | | | Blood plasma?? |
| v64 | F11a-SERPINE1 complex assembly | Complex assembly | F11a, SERPING1 | F11a-SERPING1 | | Blood plasma?? |
| v65 | F12a-SERPING1 complex assembly | Complex assembly | F12a, SERPING1 | F12a-SERPING1 | | Blood plasma?? |
| v66 | KLKB1-SERPING1 complex assembly | Complex assembly | KLKB1a, SERPING1 | KLKB1a-SERPING1 | | Blood plasma?? |
| v67 | F2a-α2-M complex assembly | Complex assembly | F2a, α2-M | F2a-α2-M | | |
| v68 | Substrate catalysis | Catalysis | subs | subsa | F2a | |
| v69 | Substrate catalysis | Catalysis | subs | subsa | F2a-α2-M | |

Table 2. All reaction mechanisms incorporated in the computer model of the fibrin polymerization.

| Reaction | Name | Type | Substrates | Products | Cofactors/ Catalyst | Reaction site |
|---|---|---|---|---|---|---|
| v70 | FpA cleavage from Fg | Catalysis | Fg | desAA-Fg, 2 FpA | F2a | Blood plasma |
| v71 | FpB cleavage from Fg | Catalysis | Fg | desBB-Fg, 2 FpB | F2a | Blood plasma |
| v72 | FpA cleavage from desAA-Fg | Catalysis | desAA-Fg | Fn, 2 FpA | F2a | Blood plasma |
| v73 | FpB cleavage from desBB-Fg | Catalysis | desBB-Fg | Fn, 2 FpB | F2a | Blood plasma |
| v74 | FpA cleavage from Fg-F2a | Catalysis | Fg-F2a | desAA-Fg-F2a | F2a | Blood plasma |
| v75 | FpB cleavage from Fg-F2a | Catalysis | Fg-F2a | desBB-Fg-F2a | F2a | Blood plasma |
| v76 | Protofibril formation/ growth | Complex assembly* | $P_nP_m$ | $P_{n+m}$ | | Blood plasma |
| v77 | Fiber formation/ growth | Complex assembly** | $F_o$, $F_p$ | $F_{n+m}$ | Fn | Blood plasma |

*$P_n + P_m \rightarrow P_{n+m} \forall n+m \leq 29, n>0, m>0$
**$F_o + F_p \rightarrow P_{n+m} \forall o+p \leq 9, o > 0, p>0$

Table 3. All reaction mechanisms incorporated in the computer model regarding the effect of unfractionated heparin (UFH) and low-molecular weight heparin (LMWH) on the function of AT-III. It should be noted that in this table the official gene symbol of AT-III (SERPINC1) is used instead of the popular scientific names.

| Reaction | Name | Type | Substrates | Products | Cofactors/ Catalyst | Reaction site |
|---|---|---|---|---|---|---|
| v78 | SERPINC1-UFH complex assembly | Complex assembly | SERPINC1, UFH | SERPINC1-UFH | | Blood plasma |
| v79 | F11a-SERPINC1-UFH complex assembly | Complex assembly | F11a, SERPINC1-UFH | F11a-SERPINC1-UFH | | Blood plasma |
| v80 | F9a-SERPINC1-UFH complex assembly | Complex assembly | F9a, SERPINC1-UFH | F9a-SERPINC1-UFH | | Blood plasma |
| v81 | F2a-SERPINC1-UFH complex assembly | Complex assembly | F2a, SERPINC1-UFH | F2a-SERPINC1-UFH | | Blood plasma |
| v82 | F10a-SERPINC1-UFH complex assembly | Complex assembly | F10a, SERPINC1-UFH | F10a-SERPINC1-UFH | | Blood plasma |

(continued)

| Reaction | Name | Type | Substrates | Products | Cofactors/ Catalyst | Reaction site |
|---|---|---|---|---|---|---|
| v83 | F3-F7a-SERPINC1-UFH complex assembly | Complex assembly | F3-F7a, SERPINC1-UFH | F3-F7a-SERPINC1-UFH | | Blood plasma |
| v84 | F10a-F5a-SERPINC1-UFH complex assembly | Complex assembly | F10a-F5a, SERPINC1-UFH | F10a-F5a-SERPINC1-UFH | | Blood plasma |
| v85 | SIRPINC1-LMWH complex assembly | Complex assembly | SERPINC1, LMWH | SERPINC1-LMWH | | Blood plasma |
| v86 | F11a-SERPINC1-LMWH complex assembly | Complex assembly | F11a, SERPINC1-LMWH | F11a-SERPINC1-LMWH | | Blood plasma |
| v87 | F9a-SERPINC1-LMWH complex assembly | Complex assembly | F9a, SERPINC1-LMWH | F9a-SERPINC1-LMWH | | Blood plasma |
| v88 | F2a-SERPINC1-LMWH complex assembly | Complex assembly | F2a, SERPINC1-LMWH | F2a-SERPINC1-LMWH | | Blood plasma |
| v89 | F10a-SERPINC1-LMWH complex assembly | Complex assembly | F10a, SERPINC1-LMWH | F10a-SERPINC1-LMWH | | Blood plasma |
| v90 | F3-F7a-SERPINC1-LMWH complex assembly | Complex assembly | F3-F7a, SERPINC1-LMWH | F3-F7a-SERPINC1-LMWH | | Blood plasma |
| v91 | F10a-F5a-SERPINC1-LMWH complex assembly | Complex assembly | F10a-F5a, SERPINC1-LMWH | F10a-F5a-SERPINC1-LMWH | | Blood plasma |

Table 4. The ordinary differential equations incorporated in the computer model regarding, the PK effect of unfonctionated heparin (UFH) and low molecular weiigh heparin (LMWH) All states are described on more detail in the column all other entity state equation (e.g UFH) LMWH are model constant

| Reaction | Name | Equation | Description |
|---|---|---|---|
| PKPD1 | UFH in blood compartment | $\frac{d[UFH]}{dt} = \frac{IV}{Vd_{UFH}} - k_{eUFH}[UFH]$ | [UFH] concentration of UFH in blood compartment |

(continued)

| Reaction | Name | Equation | Description |
|---|---|---|---|
| PKPD2 | LMWH in absorption compartment | $$\frac{dA_{LMWH}}{dt} = -k_a\,A_{LMWH}$$ | $A_{LMAH}$ amount of LMWH in absorption compartment |
| PKPD3 | LMWH in blood compartment | $$\frac{d[LMWH]}{dt} = k_a\frac{A_{LMWH}}{Vc_{LMWH}} - k_{12\,LMWH}\,[LMWH] \\ + k_{21\,LMWH}\frac{A_{LMWH\,P}}{Vc_{LMWH}} \\ - k_{eLMWH}\,[LMWH]$$ | [LMWH] concentration of LMWH in blood compartment |
| PKPD4 | LMWH in peripheral compartment | $$\frac{dA_{LMWH\,P}}{dt} = k_{12\,LMWH}\,[LMWH]\,Vc_{LMWH} \\ - k_{21\,LMWH}\,A_{LMWH\,P}$$ | $A_{LMWHP}$ amount of LMWH in peripheral compartment |

As mentioned, the pharmacodynamical modeling and/or pharmacokinetic modeling may not be used in some embodiments of the invention, if the user desires to do so. Compared to embodiments which use models that involve pharmacodynamical modeling and/or pharmacokinetic modeling, the embodiments avoiding such usage do not need to calculate certain aspects, as no drug distribution and interaction with the body is necessary. Therefore, said embodiments are faster in providing the user with the necessary information as said embodiments including PK/PD modeling.

Furthermore, the numerical model used in said embodiments which avoid said usage of PK/PD modeling may work on a shorter time scale, i.e. minutes rather than days.

When including the countermeasures, specifically relating to the administration of pro- and anti-coagulants it might be an advantage to provide for PK/PD modeling. Administrations may be directly into the bloodstream, so the part of PK/PD modeling that represents uptake by the body, digestion by the liver etc may not be necessary as one can simply model it as a direct increase of the concentration of the drug in the blood and time scales are indeed much shorter. Interaction of a drug with the other blood proteins is however required if the user wants to predict the effect of a countermeasure involving a drug. The present invention matches theses needs.

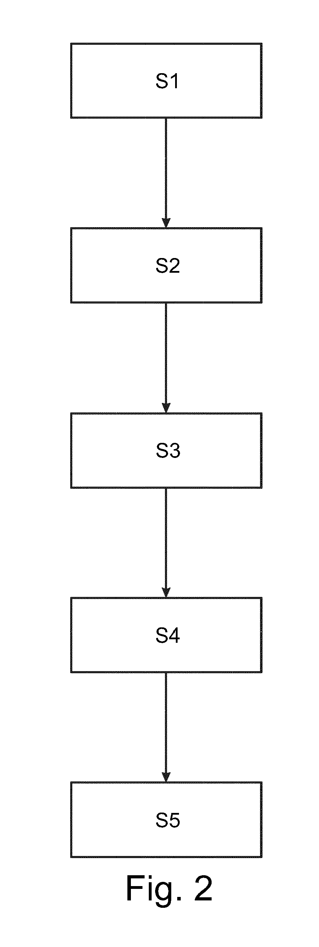

Fig. 2 is a flow diagram of a method of predicting a blood dilution risk value of a first blood circulation, wherein the presented method comprises the steps S1 to S5. Providing measured coagulation data describing the hemostatic situation of a first blood circulation at a first point in time is depicted as step S1. Furthermore, applying the measured coagulation data as an input for a numerical model is presented as step S2, wherein the numerical model is mathematical and dynamical representation of a blood dilution situation of the first blood circulation. Moreover, in step S3, a simulation of a time development of the hemostatic situation by means of the numerical model and based on the measured coagulation data used as an input for the numerical model is performed. In step S4, a calculation of values of concentrations of human blood proteins as an output of the simulation is performed. The step S5 describes the translating of at least some of the calculated values of the concentrations of the human blood proteins into a risk value, which risk value describes a risk of clotting and/or embolism and/or bleeding for the first blood circulation.

To perform the presented method a CDS system may be used that incorporates all of the monitored and additional patient data and uses this to provide an early warning when the patient's hemostatic level starts to move into the danger zone, and provides advice on the optimal countermeasure to regain hemostatic balance. The solution uses a computer model that takes into account the patient's data and the estimated dilution of the blood (through blood loss and transfusions) and predicts when the patient's hemostatic balance shifts to a dangerous level. The same model can simulate the expected effect of a number of procedures that are designed to regain hemostatic balance, and recommend the procedure with the highest chance of success. The model may make its calculation near instantaneously, before e.g. a next test is performed. This may provide the anaesthesiologist with the opportunity to keep the patient within the stable hemostatic range, instead of returning the patient to safety after (s)he has left the stable range. If the patient should stray outside the stable range after all, the model can estimate the expected results for each of a set of available countermeasures. This will allow the anaesthesiologist to choose the optimal countermeasure, performed in the optimal way, and thus to stabilize the patient as fast as possible.

The aforementioned model used in the embodiment of Fig. 2 may be implemented as a differential equation model that describes inter alia the interactions of coagulation proteins and formation and break-down of the thrombus. Such model is a dynamic model, as it describes the evolution of system states like protein concentrations or thrombus size over time. The time dynamics of each interaction mechanism is described as an ordinary differential equation or ODE that involves the concentration(s) of the protein(s) and/or chemical molecule(s) that are involved in the reaction and the reaction rate parameter(s). By summation of all reaction mechanisms in which a particular protein or other kind of chemical molecular is involved (a protein or molecule can participate in more than one reaction), the time dynamics of the concentration of that particular protein or other kind of chemical entity is calculated. The whole system can be calculated by keeping track of the evolution of all proteins and molecules.

As model parameters, the initial conditions of the system, i.e. the concentration of all proteins and molecules at t = 0 and the reaction rate parameters of the reaction mechanisms may be entered into the model by a user. Part of the initial concentrations may also be measured in a laboratory or a clinic, whereas others may be taken from literature, i.e. average patient values, possibly corrected for gender and age. The reaction rate parameters may be derived via solving an inverse problem, i.e. model fitting to experimental data. The system of ODEs may be solved numerically, using the numerical values of the model parameters, by employing ODE integration algorithms.

The method of Fig. 2 may comprise the step of automatically suggesting an application for administration of a coagulant and/or an anti-coagulant.

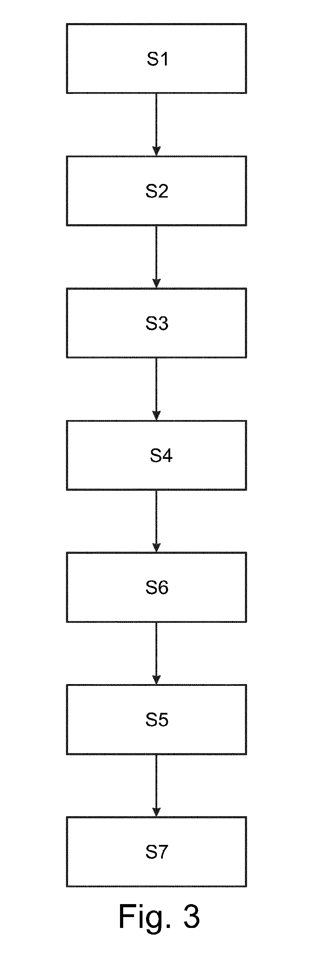

Fig. 3 shows another exemplary embodiment of a method of predicting a hemostatic situation of a first blood circulation by means of a flow diagram. With regard to steps S 1 to S4 and S5, it is referred to the presented disclosure and description of Fig. 2. However, compared to Fig. 2, the embodiment of Fig. 3 provides a step S6. The method of Fig. 3 specifies that the calculated values of the concentrations of the human blood proteins are m values of k different proteins. The step S6 defines to choose n values out of the m values. Therein, k, m and n are integers and n < m. Furthermore, only the n values are taken into account for the translation into the risk value which is performed in step S5. Moreover, the method of Fig. 3 defines to graphically display a time development of the risk value on a graphical user interface within step S7. If desired, additional information may be stored on for example the storing arrangement 103 of Fig. 1, which information is used by the presented method in order to decide which n values out of the m values are chosen. For example, only the most sensitive types of human blood proteins are chosen out of the proteins for which concentrations have been calculated. However, also other criteria may be applied.

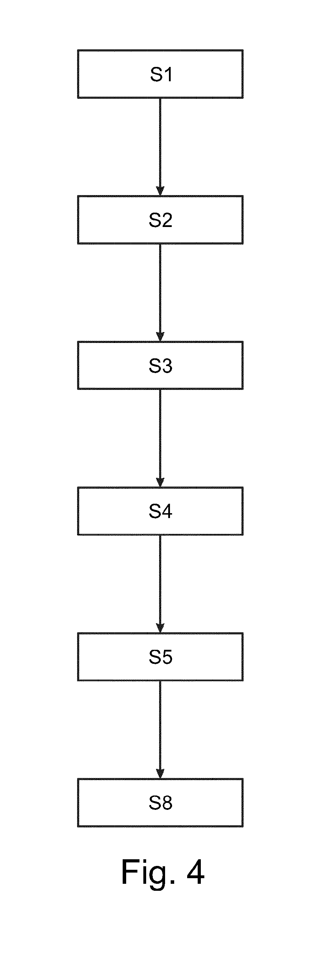

Fig. 4 describes another exemplary embodiment of a method of predicting a hemostatic situation of a first blood circulation according to an embodiment of the present invention. Regarding steps S1 to S5, it is kindly referred to the description and disclosure of Fig. 2. Additionally, step S8 is comprised by the method of Fig. 4. The step of using the numerical model to identify a set of most sensitive state variables in a situation of blood dilution based on at least one given sensitivity threshold is depicted by step S8. Details additional about such an identification have been described already above and said details may be integrated in the embodiment of Fig. 4.

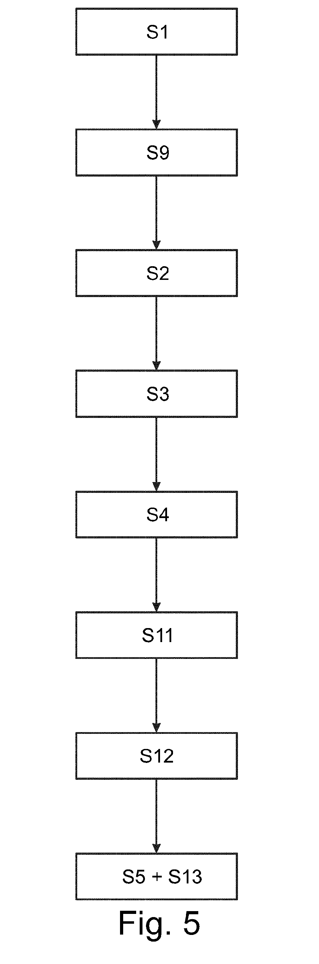

Fig. 5 shows another exemplary embodiment of a method according to the present invention. Providing measured coagulation data which describe the hemostatic situation of the first blood circulation at a first point in time is depicted with step S1. Additionally, data about human blood protein levels of a second blood circulation at a second point in time as reference protein levels are provided, which provision is depicted by step S9. The first and the second blood circulation may be the same, which would lead to a specific observation of one patient. Furthermore, the second point in time is before the first point in time and the blood circulation undergoes bleeding and/or clotting and/or embolism at the second point in time. Therefore, it can be assured that the used reference protein levels indicate realistic levels of the corresponding proteins that occur during a bleeding and/or clotting and/or embolism. However, if desired, the first and the second blood circulation may be different, which means that reference values are retrieved from another patient. Regarding steps S2 to S4, it is kindly referred to the previously presented description of Fig. 2. Furthermore, comparing said provided reference protein levels with the calculated values of concentrations of human blood proteins is depicted in Fig. 5 by step S11. Generating a comparison value, S12, and using the comparison value for the translation into the blood dilution risk value for the first blood circulation, S 13, complete the presented method. There is to be noted that step S5 is performed in this embodiment in such a way that the risk value is calculated based on the comparison value was previously generated.

Fig. 6 shows another flow diagram of a method according to an exemplary embodiment of the invention. Regarding steps S1 to S4, it is kindly referred to the disclosure of previously described Fig. 2. As can be seen in Fig. 6, three different possibilities of how to proceed during or after step S4 are presented by Fig. 6. In detail, Fig. 6 discloses three different embodiments of the present invention. The flow diagram of Fig. 6 describes that at least some of the predicted values of the concentrations of the human blood proteins are translated into the risk value by either calculating a speed of sealing of a hypothetical wound, step S 14a, or by calculating an extent of growth of a hypothetical thrombus (i.e. the resulting thrombus size), step S 14b, as an output. Fig. 6 depicts, that firstly only the aspect of the speed of sealing of the hypothetical wound can be calculated. In such a case, the user would choose to use the left branch of Fig. 6. In case he is interested in calculating a size of the hypothetical thrombus, step S 14b, he would choose the centered branch of Fig. 6. However, if the user desires to be provided with results of both calculations, he may choose the right branch of Fig. 6. In any of the three presented branches, based on the previously calculations and based on the generated corresponding outputs, a translation into the risk value is performed, which risk value describes a risk of clotting and/or embolism and/or bleeding for the first blood circulation. This is described by the corresponding step S5.

In other words, the embodiment of Fig. 6 comprises a model which is used to simulate the growth of the thrombus or sealing of a hypothetical wound itself. If the model predicts uncontrolled growth of the thrombus, to the point where it occludes a vessel or may break into circulating pieces, and if no countermeasure is taken, the patient stability score or risk value will exceed one threshold of the save zone. In case the model of Fig. 6 predicts a sealing of the wound which is so slow that blood loss grows to a dangerous level, the save zone has been left on the other side. However, the present invention provides for improvements of such situations, as an alert may be applied to the user. Alternatively counter measures may be suggested or at least a prediction of how the risk value will evolve is supplied to the user.

If desired, any of the three embodiments of Fig. 6 may comprise the additional step of evaluating the simulated time development of the hypothetical sealing of the wound by evaluating a time that passes between an initialization of the clotting process, i. e. a formation of the wound, and a cessation of the clotting process, i. e. a sealing of the wound.

If desired, any of the embodiments described within Fig. 6, may additionally comprise a step of evaluating the risk of occlusion of a blood vessel by evaluating a size of the thrombus. This may be expressed e. g. as the total mass of fibrin present in the thrombus, the volume of the thrombus or the thickness of the thrombus, i. e. the minimum or maximum distance between the wound and/or blood vessel wall and the outer edge of the thrombus.

Additionally if desired, any of the three embodiments of Fig. 6 may comprise the additional step of evaluating the risk of embolism by evaluating a constitution of the thrombus. This may for example be embodied through the calculation of the thrombus' mass density or the mass-length ratio of the comprising fibrin fibers for the purpose of risk of embolism, caused by pieces breaking off of the main thrombus.

Fig. 7 shows a clinical decision support system according to an exemplary embodiment of the invention. In the following, clinical decision support system will be described by the shown basic structure of Fig. 7; however, it will become apparent that such a clinical decision support system may comprise many additional optional features. In general the clinical decision support system of Fig. 7 provides a solution to the above identified problems of the prior art and may comprise of a content delivery system that may incorporate all of the measured and monitored and, if desired, additional patient data and may use this to provide an early warning when the patient's hemostatic level starts to move into the danger zone. Furthermore the clinical decision support system of Fig. 7 may provide for an advice on the optimal countermeasure to a user such hemostatic balance is regained. The clinical decision support system makes use of or comprises a computer model that takes into account the patient's data and the estimated dilution of the blood through e.g. blood loss and e.g. transfusions. Furthermore the computer model may be configured to predict when the patient's hemostatic balance shifts to a dangerous level. The same model may be used to simulate the expected effect of a number of procedures i.e. countermeasures that are designed to regain hemostatic balance, and may further recommend the procedure i.e. countermeasure with the highest chance of success to the user.

One advantage of the use of the computer model according to the exemplary embodiment of Fig. 7 may be seen in that it can calculate e.g. the effect of the administration of the next unit of transfusion fluid and subsequent dilution of the blood in terms of existing diagnostic values like INR or thrombo-elastometry measurement outputs. The model can make this calculation near instantaneously, before the unit is actually administered, and before the next test is performed. This provides the anesthesiologist with the opportunity to keep the patient within the stable hemostatic range, instead of returning the patient to safety after the patient has left the stable range. If the patient should stray outside the stable range after all, the model can estimate the expected results for each of a set of available countermeasures. This will allow the anaesthesiologist to choose the optimal countermeasure, performed in the optimal way, and thus to stabilize the patient as fast as possible.

Regarding the computer model of this exemplary embodiment of Fig. 7 the following should be noted. The aforementioned model may be implemented as a differential equation model that describes e. g. the interactions of coagulation proteins, formation and break-down of the thrombus, the effect of anti-coagulants like Heparin, etc. Said model may be seen as a dynamic model which describes the evolution of states variables of the model like e.g. protein concentrations or e.g. thrombus size over time. The time dynamics of each interaction mechanism is described as an ordinary differential equation or ODE that may involve the concentration(s) of the protein(s) and/or chemical molecule (s) that are involved in the reaction and the reaction rate parameter(s). By summation of some or all reaction mechanisms in which a particular protein or other kind of chemical molecular is involved, a protein or molecule can participate in more than one reaction, the time dynamics of the concentration of that particular protein or other kind of chemical entity is calculated. The whole system can be calculated by keeping track of the evolution of some or all proteins and molecules. Thus the presented dynamic model can predict future evolution of the patient's hemostatic system based on e.g. measurements of the present.

In certain cases this may require however that besides the reaction topology, the numerical values of the model parameters are known as well. These model parameters include the initial conditions of the system, i.e. the concentration of all proteins and molecules at $t = 0$, e.g. before any blood loss, and the reaction rate parameters of the reaction mechanisms. Part of the initial concentrations may be measured in the laboratory or clinic, whereas others may be taken from literature, i.e. average patient values, that are possibly corrected for gender and age. The reaction rate parameters may be derived via solving an inverse problem, i.e. model fitting to experimental data. The system of ODEs may be solved numerically, using the numerical values of the model parameters, by employing ODE integration algorithms. The treating physician is the operator of the clinical decision support system, hence is able to give user input to the system. The other type of input can be clinical measurements, e.g. INR, aPTT, vitamin K-proteins' activity. The user interface is connected via software to the computer model; the information flow of the user interface is diverted to the computer model to serve as input. The computer model uses the given input and calculates the expected future evolution of the blood dilution of the blood circulation, which are forwarded to the user interface via the connecting software. The clinical decision support system of Fig. 7 further comprises a user interface. This exemplary embodiment of the invention may be integrated in e.g. a hospital IT system, and can be accessed via a graphical user interface on one of the computer terminals. The clinical decision support system can be provided with coagulation data about the patient that is available before instability in the haemostatic situation due to blood dilution is feared. This may be done either manually or through a retrieval of data from a data storage like e.g. a server or e.g. through a direct interface with an information system like a hospital information system or an electronic patient records. This may be seen in Fig. 8, top left panel. The clinical decision support system may interface with the monitoring and measurement devices that are used during a surgery, and/or have a simple way to enter new patient data into the system manually. This may be seen in Fig. 8, bottom left panel. One screen of the clinical decision support system should show the current risk value of the patient, e.g. visualized by a graph that plots a 'safety value' or a set of values for the patient over time and indicates when this value threatens to leave the safe zone. This may be seen in Fig. 8, top right panel. An indication of threat may be delivered to the user both in the graph and through an alarm. Such risk values can be as simple as INR or aPTT values, or a more elaborate diagnostic score value.

In more detail, Fig. 7 describes a clinical decision support system 700, which comprises a first arrangement 701, which is embodied as a user interface. Furthermore, it is shown that coagulation data 702 are supplied by means of an input 707 of a user into the user interface 701. Software 709 is shown in Fig. 7 as well as a storing arrangement 703, which simultaneously acts as a calculation arrangement 705. The numerical model 704 is stored on the storing arrangement 703. Additionally, clinical measurements or clinical data 706 may be supplied to the user interface. In combination with the following description of Fig. 8, the advantages and gist of the system will become even more apparent.

Fig. 8 shows a user interface as may be comprised by a clinical decision support system according to an exemplary embodiment of the invention. In Fig. 8 the top right panel shows both the observed risk values, shown in the left zone 802 and the predicted risk values in the right zone 803 for the monitored risk value. The observed risk values can be calculated by the numerical model based on the provided coagulation data which are provided via the bottom-left panel. Moreover, the numerical model can predict a risk value based on calculated predictions of the concentrations of the participating proteins. Upon prediction of leaving the safe zone 805 the clinical decision support system may display one or a number of countermeasures 807 to 810 to the screen, sorted by their probability of success and paired to a preference score indicating the system's preference for each measure, see bottom right panel of Fig. 8. It should be noted that this example and illustration is meant to explain the type of graphical user interface that may be used in accordance with the present invention, and is in no way exhaustive in e.g. the list of possible test values to enter or recommended actions to output. Additionally, the time development of risk value 801 is depicted in the graphical user interface 800. The separation between the area 802 describing the observed risk values and the area 803 of the predicted risk values can clearly be gathered from the top right panel of Fig. 8. In other words, the clinical decision support system is configured to provide for a calculated risk value based on measured coagulation data, and the clinical decision support system is additionally configured to provide for a predicted risk value based on simulation.

As can be seen in Fig. 8 the graphical representation of risk value 801 is displayed in an x and y diagram which provides on the y-axes a stable zone 805 detailed as an area which the risk value may develop without causing an alert. Furthermore, a danger zone 804, i. e. a danger area is shown in Fig. 8. An alert sign 806 is comprised by the graphical user interface. Furthermore, personal data about the patient can be received from external or internal databases and shown on the top left panel. Personal information 814 is displayed to the user. By means of access button 813, a connection to a database may be established. Furthermore, measured coagulation data 102 can be received by the graphical user interface via the bottom left panel. By means of button 812, this received and measured coagulation data may be submitted to the underlying numerical model in order to perform the desired predictions. Measured coagulation data may be provided to the receiving arrangement 101. Exemplarily values of a ROTEM tests are shown, which is a brand name. In general this may be called a value of a thrombo-elastometry test.

## Claims

1. Method of predicting a blood dilution risk value of a first blood circulation, the method comprising the steps:

   providing measured coagulation data describing a haemostatic situation of the first blood circulation at a first point in time (S 1),
   applying the measured coagulation data as an input for a numerical model, the numerical model being a mathematical and dynamical representation of a blood dilution of the first blood circulation (S2),

performing a simulation of a time development of the haemostatic situation by means of the numerical model and based on the measured coagulation data used as an input for the numerical model (S3),
calculating values of concentrations of human blood proteins as an output of the simulation (S4), and
translating at least some of the calculated values of the concentrations of the human blood proteins into a risk value, which risk value describes a risk of clotting and/or embolism and/or bleeding for the first blood circulation (S5).

2.   Method according to claim 1,
wherein the calculation of the values of the concentrations of the human blood proteins is generating a time development of said concentrations of human blood proteins as the output.

3.   Method according to claim 1 or 2,
wherein the calculated values of the concentrations of the human blood proteins are m values of k different proteins, the method further comprising the step:

choosing n values out of the m values (S6),
wherein k, m and n are integers and n < m, and
wherein only the n values are taken into account for the translation into the risk value.

4.   Method according to one of claims 1 to 3, further comprising the step:

graphically displaying a calculated time development of the risk value (801) on a graphical user interface (S7).

5.   Method according to one of claims 1 to 4,
wherein the at least some of the values of concentrations of human blood proteins are translated into the risk value by means of a numerical function of state variables of the numerical model.

6.   Method according to claim 5
wherein the state variables of the numerical model are chosen from the group comprising concentrations of the following proteins: Alpha-2-Macroglobulin (A2M), C4BP, coagulation factor 10 (F10), F11, F13, prothrombin (F2), tissue factor, F5, F7, F8, F9, fibrinogen, fibrin, protein C, protein S, protein Z, protein Z related protein inhibitor (ZPI), alpha-1-anti-trypsin (AAT), protein C inhibitor (PCI), anti-thrombin (ATIII), PAI1, C1 inhibitor (Clinh), TAFI, TFPI, Vitronectin, plasmin, plasminogen, A2AP, thrombomodulin, uPA, tPA, the proteins' activated forms F10a, F11a, F13a, thrombin (F2a), F5a, F7a, F8a, F9a, activated protein C, and TAFIa, or
wherein the state variables of the numerical model comprise a concentration of complexes formed by at least two of the previously cited proteins (e.g FVa-FXa), or
wherein the state variables of the numerical model comprise a mass-length ratio of fibrin fibers formed in coagulation.

7.   Method according to one of claims 5 or 6, further comprising the step:

using the numerical model to identify a set of most sensitive state variables in a situation of blood dilution based on at least one given sensitivity threshold (S8).

8.   Method according to one of claims 1 to 7, further comprising the step:

providing data about human blood protein levels of a second blood circulation at a second point in time as reference protein levels (S9),
wherein at the second point in time the second blood circulation undergoes bleeding and/or clotting and/or embolism,
wherein the second point in time is before the first point in time, and
using said provided reference protein levels for the translation into the risk value for the first blood circulation (S 10).

9.   Method according to one of claims 1 to 8,
wherein the at least some of the values of the concentrations of the human blood proteins are translated into the risk value by calculating a speed of sealing of a hypothetical wound (S 14a) as the output or by calculating an extent of growth of a hypothetical thrombus as the output (S14b).

**10.** Method according to claim 9,
wherein the numerical model is a model of a time development of a hypothetical sealing of a wound of the first blood circulation, further comprising the steps:

performing the simulation of the time development of the hypothetical sealing of the wound of the first blood circulation in terms of at least one element of the group comprising: a wound surface area, interaction of tissue factors in a wound surface area with coagulation proteins in the first blood circulation, formation of fibrin fibers, and aggregation of blood platelets and/or fibrin fibers, which cover a wound surface and stop a clotting process.

**11.** Method according to claim 10, further comprising the steps:

evaluating the simulated time development of the hypothetical sealing of the wound by evaluating a time that passes between an initialization of the clotting process, i.e. a formation of the wound, and an cessation of the clotting process, i.e. a sealing of the wound, and/or

**12.** Method according to one of claims 1 to 11, further comprising the steps:

calculating a size and/or constitution of a thrombus of the first blood circulation, and
evaluating a risk value based on the size and/or the constitution of the thrombus.

**13.** Clinical decision support system (100) for predicting and displaying a blood dilution risk value of a first blood circulation, the system comprising:

a first arrangement (101) configured to receive measured coagulation data (102) describing a haemostatic situation of the first blood circulation at a first point in time,
a storing arrangement (103) on which a numerical model (104) is stored, wherein the numerical model is a mathematical and dynamical representation of a blood dilution of the first blood circulation,
a calculation arrangement (105) configured to perform a simulation of a time development of the haemostatic situation by means of the numerical model and based on the measured coagulation data used as an input for the numerical model,
wherein the calculation arrangement is configured to calculate values of concentrations of human blood proteins as an output of the simulation, and
wherein the calculation arrangement is configured to translate at least some of the calculated values of concentrations of human blood proteins into a risk value, which risk value describes a risk of clotting and/or embolism and/or bleeding of the first blood circulation, further comprising
a display arrangement (106) configured to display the risk value.

**14.** Program element (107) for predicting and displaying a blood dilution risk value of a first blood circulation, which when being executed by a processor (108) is adapted to carry out:

receiving measured coagulation data describing a haemostatic situation of the first blood circulation at a first point in time (S1a),
applying the measured coagulation data as an input for a numerical model, the numerical model being a mathematical and dynamical representation of a blood dilution of the first blood circulation (S2),
performing a simulation of a time development of the haemostatic situation by means of the numerical model and based on the measured coagulation data used as an input for the numerical model (S3),
calculating values of concentrations of human blood proteins as an output of the simulation (S4), and
translating at least some of the calculated values of concentrations of human blood proteins into a risk value, which risk value describes a risk of clotting and/or embolism and/or bleeding for the first blood circulation (S5).

**15.** Computer readable medium (109) in which a program element (107) for predicting and displaying a blood dilution risk value of a first blood circulation is stored, which, when being executed by a processor (108) is adapted to carry out:

receiving measured coagulation data describing a haemostatic situation of the first blood circulation at a first point in time (S1a),
applying the measured coagulation data as an input for a numerical model, the numerical model being a mathematical and dynamical representation of a blood dilution of the first blood circulation (S2),
performing a simulation of a time development of the haemostatic situation by means of the numerical model

and based on the measured coagulation data used as an input for the numerical model (S3),
calculating values of concentrations of human blood proteins as an output of the simulation (S4), and
translating at least some of the calculated values of concentrations of human blood proteins into a risk value,
which risk value describes a risk of clotting and/or embolism and/or bleeding for the first blood circulation (S5).

Fig. 1

```
┌─────────────────┐
│                 │
│       S1        │
│                 │
└─────────────────┘
         │
         ▼
┌─────────────────┐
│                 │
│       S2        │
│                 │
└─────────────────┘
         │
         ▼
┌─────────────────┐
│                 │
│       S3        │
│                 │
└─────────────────┘
         │
         ▼
┌─────────────────┐
│                 │
│       S4        │
│                 │
└─────────────────┘
         │
         ▼
┌─────────────────┐
│                 │
│       S5        │
│                 │
└─────────────────┘
```

Fig. 2

Fig. 3

```
┌─────────────────┐
│       S1        │
└─────────────────┘
         │
         ▼
┌─────────────────┐
│       S2        │
└─────────────────┘
         │
         ▼
┌─────────────────┐
│       S3        │
└─────────────────┘
         │
         ▼
┌─────────────────┐
│       S4        │
└─────────────────┘
         │
         ▼
┌─────────────────┐
│       S5        │
└─────────────────┘
         │
         ▼
┌─────────────────┐
│       S8        │
└─────────────────┘
```

Fig. 4

```
┌─────────────────┐
│       S1        │
└─────────────────┘
         │
         ▼
┌─────────────────┐
│       S9        │
└─────────────────┘
         │
         ▼
┌─────────────────┐
│       S2        │
└─────────────────┘
         │
         ▼
┌─────────────────┐
│       S3        │
└─────────────────┘
         │
         ▼
┌─────────────────┐
│       S4        │
└─────────────────┘
         │
         ▼
┌─────────────────┐
│       S11       │
└─────────────────┘
         │
         ▼
┌─────────────────┐
│       S12       │
└─────────────────┘
         │
         ▼
┌─────────────────┐
│    S5 + S13     │
└─────────────────┘
```

Fig. 5

Fig. 6

700

706                                              702

707

701

708

709

710

703

704

705

Fig. 7

# EUROPEAN SEARCH REPORT

Application Number

EP 11 18 8547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | "THE QUICK MACHINE - A MATHEMATICAL MODEL FOR THE EXTRINSIC ACTIVATION OF COAGULATION", HAEMOSTASIS, BASEL, CH, vol. 24, 1 January 1994 (1994-01-01), pages 325-337, XP002928227, ISSN: 0301-0147 * the whole document * | 1-15 | INV. G06F19 |
| X | L. J. ENRIQUEZ ET AL: "Point-of-care coagulation testing and transfusion algorithms", BRITISH JOURNAL OF ANAESTHESIA, vol. 103, no. Supplement 1, 1 December 2009 (2009-12-01), pages i14-i22, XP055039793, ISSN: 0007-0912, DOI: 10.1093/bja/aep318 * the whole document * | 1-15 | |
| X | MICHAEL T. GANTER ET AL: "Coagulation Monitoring: Current Techniques and Clinical Use of Viscoelastic Point-of-Care Coagulation Devices", ANESTHESIA & ANALGESIA, vol. 106, no. 5, 1 May 2008 (2008-05-01), pages 1366-1375, XP055039818, ISSN: 0003-2999, DOI: 10.1213/ane.0b013e318168b367 * the whole document * | 1-15 | TECHNICAL FIELDS SEARCHED (IPC) G01N G06F |
| X | US 6 890 299 B2 (COHEN ELI [US] ET AL) 10 May 2005 (2005-05-10) * claim 1 * * column 6, line 44 - line 64 * * column 8, line 15 - line 22 * | 1-15 | |

-/--

The present search report has been drawn up for all claims

| Place of search | Date of completion of the search | Examiner |
|---|---|---|
| Munich | 4 October 2012 | Sisk, Aisling |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 11 18 8547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 321 164 B1 (BRAUN PAUL [US] ET AL) 20 November 2001 (2001-11-20) * column 2, line 47 - line 56 * * column 3, line 15 - line 26 * * column 3, line 42 - line 62 * * column 5, line 23 - line 46 * * column 6, line 11 - line 17 * ----- | 1-15 | |
| X | KUCHER N ET AL: "Electronic Alerts to Prevent Venous Thromboembolism Among Hospitalized Patients", ACC CURRENT JOURNAL REVIEW, ELSEVIER, NL, vol. 14, no. 8, 1 August 2005 (2005-08-01), page 19, XP027656638, ISSN: 1062-1458 [retrieved on 2005-08-01] * the whole document * ----- | 1-15 | |
| X | A. H. STAMMERS ET AL: "Point-of-care coagulation monitoring: applications of the thromboelastograph", ANAESTHESIA, vol. 53, no. S2, 1 May 1998 (1998-05-01), pages 58-59, XP055039832, ISSN: 0003-2409, DOI: 10.1111/j.1365-2044.1998.tb15159.x * the whole document * ----- | 1-15 | TECHNICAL FIELDS SEARCHED (IPC) |
| X | US 6 730 026 B2 (CHRIST TILO [DE] ET AL) 4 May 2004 (2004-05-04) * the whole document * ----- | 1-15 | |

The present search report has been drawn up for all claims

| Place of search | Date of completion of the search | Examiner |
|---|---|---|
| Munich | 4 October 2012 | Sisk, Aisling |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 18 8547

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2012

| Patent document cited in search report | | Publication date | Patent family member(s) | | Publication date |
|---|---|---|---|---|---|
| US 6890299 | B2 | 10-05-2005 | EP | 1611444 A1 | 04-01-2006 |
| | | | US | 2004203163 A1 | 14-10-2004 |
| | | | WO | 2004092742 A1 | 28-10-2004 |
| US 6321164 | B1 | 20-11-2001 | AU | 1950399 A | 19-07-1999 |
| | | | CA | 2316361 A1 | 08-07-1999 |
| | | | EP | 1042669 A1 | 11-10-2000 |
| | | | JP | 2002500360 A | 08-01-2002 |
| | | | US | 6321164 B1 | 20-11-2001 |
| | | | US | 2002019706 A1 | 14-02-2002 |
| | | | WO | 9934208 A1 | 08-07-1999 |
| US 6730026 | B2 | 04-05-2004 | DE | 10103330 A1 | 14-08-2002 |
| | | | EP | 1226781 A2 | 31-07-2002 |
| | | | US | 2002099283 A1 | 25-07-2002 |

EPO FORM P0459

For more details about this annex : see Official Journal of the European Patent Office, No. 12/82

**EP 2 538 360 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **FREY, H.C. ; PATIL, S. R.** Identification and Review of Sensitivity Analysis Methods. *Risk Anal.,* 2002, vol. 22 (3), 553-578